(19) Europäisches Patentamt
European Patent Office
Office européen des brevets

(11) Veröffentlichungsnummer: **0 030 215**
**A2**

(12) # EUROPÄISCHE PATENTANMELDUNG

(21) Anmeldenummer: **80810365.9**

(22) Anmeldetag: **24.11.80**

(51) Int. Cl.$^3$: **C 07 D 211/88**
C 07 D 213/61
//C07C121/16, C07C121/20

(30) Priorität: **30.11.79 CH 10661/79**

(43) Veröffentlichungstag der Anmeldung:
**10.06.81 Patentblatt 81/23**

(84) Benannte Vertragsstaaten:
**AT BE CH DE FR GB IT LI NL**

(71) Anmelder: **CIBA-GEIGY AG**
**Patentabteilung Postfach**
**CH-4002 Basel(CH)**

(72) Erfinder: **Martin, Pierre, Dr.**
**Meisenweg 38**
**CH-4310 Rheinfelden(CH)**

(72) Erfinder: **Bellus, Daniel, Dr.**
**Unterm Schellenberg 81**
**CH-4125 Riehen(CH)**

(54) **3,3,5-Trichlorglutarsäureimid, Verfahren zu dessen Herstellung und dessen Verwendung zur Herstellung von 2,3,5,6-Tetrachlorpyridin.**

(57) 3,3,5-Trichlorglutarsäureimid kann dadurch hergestellt werden, dass man entweder einen Trichloressigsäurealkylester in Gegenwart eines Katalysators mit Acrylnitril zum entsprechenden 2,2,4-Trichlor-4-cyanobuttersäurealkylester umsetzt, diesen in das Amid überführt und das 2,2,4-Trichlor-4-cyano-butancarbonsäureamid in wässrigem saurem Medium zum 3,3,5-Trichlorglutarsäureimid cyclisiert oder indem man Trichloracetonitril in Gegenwart eines Katalysators zum 2,2,4-Trichlor-4-cyanobutyronitril umsetzt und dieses in wässrigem saurem Medium zum 3,3,5-Trichlorglutarsäureimid cyclisiert. Als Katalysatoren für die Anlagerungsreaktionen können z.B. Kupfer(I)-chlorid oder Kupfer(II)oxid verwendet werden. Das 3,3,5-Trichlorglutarsäureimid lässt sich durch Behandlung mit dehydratisierenden Chlorierungsmitteln, wie $POCl_3$, in das bekannte 2,3,5,6-Tetrachlorpyridin überführen, das seinerseits Anwendung zur Herstellung verschiedener Wirkstoffe, besonders von Insektiziden, Herbiziden und Fungiziden, findet.

EP 0 030 215 A2

5-12615/ZFO/=

3,3,5-Trichlorglutarsäureimid,Verfahren zu dessen Herstellung und dessen Verwendung zur Herstellung von 2,3,5,6-Tetrachlorpyridin

Die vorliegende Erfindung betrifft 3,3,5-Trichlorglutarsäureimid als neue Verbindung, Verfahren zu dessen Herstellung und dessen Verwendung zur Herstellung von 2,3,5,6-Tetrachlorpyridin.

2,3,5,6-Tetrachlorpyridin und das daraus durch Hydrolyse erhältliche 3,5,6-Trichlorpyridin-2-ol stellen wertvolle Zwischenprodukte zur Herstellung verschiedenartiger Wirkstoffe, vor allem von Insektiziden, Herbiziden und Fungiziden dar [vgl. z.B. US Patentschriften 4.133.675, 3.244.586, 3.355.278 und 3.571.421; französische Patentschrift 2.171.939 sowie J.Agr. Food Chem. 14,304 (1966)]. Die bisher bekanntgewordenen Verfahren zur Herstellung von 2,3,5,6-Tetrachlorpyridin sind in verschiedener Hinsicht unbefriedigend. 2,3,5,6-Tetrachlorpyridin kann durch Hochtemperatur-Chlorierung von Pyridin bzw. Pyridinderivaten,wie 3,5-Dichlor-2-trichlormethylpyridin und 2,6-Dichlorpyridin (ca. 200-600°, bevorzugt etwa 350-600° C), durch Umsetzung von Glutarsäuredinitril mit Chlor in der Gasphase bei erhöhten Temperaturen (ca. 400-600° C) oder auch durch Chlorierung von $\varepsilon$-Caprolactam oder Cyclohexanonoxim bei erhöhten Temperaturen erhalten werden. Dabei entstehen jedoch neben dem gewünschten symmetrischen Tetrachlorpyridin eine Reihe anderer hochchlorierter Pyridine, die abgetrennt werden müssen (vgl. z.B. die britischen Patentschriften 1.050.378, 1.334.922 und 991.526, die US Patentschriften 3.420.833 und 3.538.100 sowie die deutschen Offenlegungsschriften 1.445.683 und 2.141.632).

2,3,5,6-Tetrachlorpyridin kann auch durch stufenweise Chlorierung von 6-Brom- oder 6-Chlor-2-äthoxypyridin erhalten werden [vgl. z.B. Recueil trav.chim., 70, 187 (1951)]. Eine andere Möglichkeit besteht darin, dass man die Hochtemperaturchlorierung mit überschüssigem Chlorierungsmittel mehr oder weniger selektiv bis zum Pentachlorpyridin führt und anschliessend das in 4-Stellung befindliche Chloratom entweder mit Zink (vgl. z.B. US Patentschrift 3.993.654) oder elektrolytisch (vgl. z.B. US Patentschrift 3.694.322) abreduziert. Bei der Reduktion mit Zink fallen grosse Mengen von Zinksalzen an, was vom ökologischen Standpunkt aus unerwünscht ist. Die Hochtemperaturchlorierung, die elektrolytische Reduktion sowie die (elektrolytische) Generation von Zink bedingen andererseits einen sehr hohen Energiekonsum. Das Pentachlorpyridin selber weist im übrigen eine relativ starke Haut und Augen reizende Wirkung auf.

Es wurde nun gefunden, dass sich unter Vermeidung der obigen Nachteile 3,3,5-Trichlorglutarsäureimid auf einfache, wirtschaftliche und umweltfreundliche Weise unter Verwendung von leicht zugänglichen, billigen Ausgangsprodukten dadurch herstellen lässt, dass man entweder

a) einen Trichloressigsäurealkylester der Formel I

$$Cl_3C\text{-}COOR \qquad (I),$$

worin R Alkyl mit 1 bis 6 Kohlenstoffatomen bedeutet, in Gegenwart eines Katalysators mit Acrylnitril zu einem 2,2,4-Trichlor-4-cyano-essigsäurealkylester der Formel II

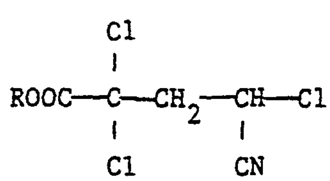

(II) ,

worin R die unter Formel I angegebene Bedeutung hat, umsetzt, die Verbindung der Formel II in Gegenwart von Ammoniak in das 2,2,4-Trichlor-4-cyano-butancarbonsäureamid überführt und das 2,2,4-Trichlor-4-cyano-butancarbonsäureamid in wässrigem saurem Medium zum 3,3,5-Trichlorglutarsäureimid cyclisiert oder

b) Trichloracetonitril in Gegenwart eines Katalysators mit Acrylnitril zum 2,2,4-Trichlor-4-cyanobutyronitril in wässrigem saurem Medium zum 3,3,5-Trichlorglutarsäureimid cyclisiert.

Die 2,2,4-Trichlor-4-cyanobuttersäurealkylester der Formel I, das 2,2,4-Trichlor-4-cyano-butancarbonsäureamid und das 2,2,4-Trichlor-4-cyanobutyronitril sind neue Verbindungen und ebenfalls Gegenstand vorliegender Erfindung.

Alkylgruppen R können geradkettig oder verzweigt sein und weisen insbesondere 1 bis 4 Kohlenstoffatome im Alkyl auf. Besonders bevorzugt verwendet man als Verbindung der Formel I den entsprechenden Essigsäuremethyl- oder Essigsäureäthylester.

Das 3,3,5-Trichlorglutarsäureimid kann anschliessend auf einfache Weise durch Behandlung mit einem dehydratisierenden Chlorierungsmittel in das 2,3,5,6-Tetrachlorpyridin übergeführt werden.

Als Katalysatoren für die Anlagerung des Trichloressigsäurealkylesters der Formel I bzw. des Trichloracetonitrils an das Acrylnitril können im erfindungsgemässen Verfahren an sich bekannte Verbindungen eingesetzt werden, wie Metalle der Hauptgruppe VIII und der Nebengruppen VIa, VIIa, Ib und IIb des Periodischen Systems (gemäss Lehrbuch der anorgan. Chemie, Holleman-Wiberg, W. de Gruyter & Co., Berlin), z.B. Eisen, Kobalt, Nickel, Ruthenium, Rhodium, Palladium, Chrom, Molybdän, Mangan, Kupfer und Zink. Diese Metalle können in elementarer Form oder in Form von Verbindungen eingesetzt werden. Geeignete derartige Verbindungen sind beispielsweise Oxide, Halogenide, Sulfate, Sulfite, Sulfide, Nitrate, Acetate, Stearate, Citrate, Carbonate, Cyanide und Rhodanide, sowie Komplexe mit Liganden, wie Phosphinen, Phosphiten, Benzoyl- und Acetylacetonaten, Nitrilen,

Isonitrilen und Kohlenmonoxid.

Als Beispiele seien genannt: Kupfer(II)oxid, Eisen(III)-oxid; Cu(I)-, Cu(II)-, Fe(II)- und Fe(III)bromide, -jodide und vor allem -chloride, Zinkchlorid, sowie die Chloride des Rutheniums, Rhodiums, Palladiums, Kobalts und Nickels; Cu(II)-sulfat, Fe(II)- und Fe(III)sulfat; Cu(II)nitrat und Eisen(III)-nitrat; Mangan(III)acetat, Kupfer(II)acetat, Kupfer(II)stearat, Eisen(III)citrat, Cu(I)cyanid; Ruthenium(II)dichloro-tris-triphenylphosphin, Rhodium-dichloro-tris-triphenylphosphin; Chrom- und Nickelacetylacetonat, Kupfer(II)acetylacetonat, Eisen(III)acetylacetonat, Kobalt(II)- und Kobalt(III)acetylacetonat, Mangan(II)acetylacetonat, Kupfer(II)benzoylacetaonat; Eisencarbonyl-cyclopentadienylkomplex; Molybdäncarbonylcyclopentadienylkomplex, Chromtricarbonylarylkomplexe, Ruthenium(II)acetatokomplex, Chrom- und Molybdänhexacarbonyl, Nickeltetracarbonyl, Eisenpentacarbonyl, Kobalt- und Mangancarbonyl.

Es können auch Gemische der genannten Metalle mit Metallverbindungen und/oder anderen Zusätzen verwendet werden, wie Kupferpulver in Kombination mit einer der vorerwähnten Kupferverbindungen; Gemische von Kupferpulver mit Lithiumhalogeniden, wie Lithiumchlorid, oder mit Isocyaniden, wie tert.-Butylisocyanid; Gemische von Eisenpulver mit Eisen(III)chlorid, gegebenenfalls unter Zusatz von Kohlenmonoxid; Gemische von Eisen(III)chlorid und Benzoin; Gemische von Eisen(II)- oder Eisen(III)chlorid und Trialkylphosphiten; Gemische von Eisenpentacarbonyl und Jod.

Bevorzugt sind Eisen(III)oxid, Eisen(II)- und Eisen(III)salze und -komplexe, vor allem Eisen(II)- und Eisen(III)chlorid, sowie Eisenpulver; Ruthenium(III)chlorid, Ruthenium(II)dichloro-tris-triphenylphosphin, Kupferpulver, Kupferbronze, Kupfer(II)oxid, Kupfer(I)- und Kupfer(II)-salze und -komplexe, wie Cu(I)chlorid, Cu(II)chlorid, Cu(I)bromid, Cu(II)bromid; Cu(II)acetat, Cu(II)acetylacetonat, Cu(II)benzoylacetonat, Cu(II)sulfat, Cu(II)nitrat,

Cu(I)cyanid und Cu(I)jodid.

Ganz besonders bevorzugt sind Kupfer(II)oxid, Kupferpulver, Kupferbronze, Kupfer(I)- und Kupfer(II)chlorid bzw. -bromid, Kupfer(I)jodid, sowie deren Gemische.

Die Katalysatoren werden im allgemeinen in Mengen von etwa 0,01 bis 10 Mol-%, bevorzugt 0,1 bis 5 Mol-%, bezogen auf das Acrylnitril, verwendet.

Die Umsetzung des Acrylnitrils mit dem Trichloressigsäurealkylester der Formel I bzw. dem Trichloracetonitril wird zweckmässig in Gegenwart eines organischen Lösungsmittels vorgenommen. Geeignete organische Lösungsmittel sind solche, in denen die Katalysatoren ausreichend löslich sind oder die mit den Katalysatoren Komplexe bilden können, die aber gegenüber der Verbindung der Formel I bzw. dem Trichloracetonitril und dem Acrylnitril inert sind. Beispiele solcher Lösungsmittel sind Alkylnitrile, besonders solche mit 2 bis 5 Kohlenstoffatomen, wie Acetonitril, Propionitril und Butyronitril; 3-Alkoxypropionitrile mit 1 oder 2 Kohlenstoffatomen im Alkoxyteil, wie 3-Methoxypropionitril und 3-Aethoxypropionitril; aromatische Nitrile, vor allem Benzonitril, sowie auch Acrylnitril (d.h. überschüssiges Reagens als Lösungsmittel); aliphatische Alkohole mit bis zu 6 Kohlenstoffatomen, wie Methanol, Aethanol, Propanol, Isopropanol, Butanole und Pentanole; aliphatische Ketone mit bevorzugt insgesamt 3 bis 8 Kohlenstoffatomen, wie Aceton, Diäthylketon, Methylisopropylketon, Diisopropylketon, Methyl-tert-butylketon;
Alkyl - und Alkoxyalkylester von aliphatischen Monocarbonsäuren mit insgesamt 2 bis 6 Kohlenstoffatomen, wie Ameisensäuremethyl- und -äthylester, Essigsäuremethyl-, -äthyl-, -n-butyl- und -isobutylester sowie 1-Acetoxy-2-methoxyäthan; cyclische Aether, wie Tetrahydrofuran, Tetrahydropyran und Dioxan; N,N-Dialkylamide von aliphatischen Monocarbonsäuren mit 1 bis 3 Kohlenstoffatomen im Säureteil, wie N,N-Dimethylformamid, N,N-Dimethylacetamid, N,N-Diäthylacetamid und N,N-Dimethylmethoxyacetamid; Aethylenglykol- und Diäthylenglykol-dialkyläther mit je 1 bis 4 Kohlenstoffatomen in den Alkylteilen, wie Aethylenglykoldimethyl-, -diäthyl- und -di-n-butyläther; Diäthylenglykoldiäthyl- und -di-n-butyläther; Hexamethylphosphorsäuretriamid (Hexametapol).

Besonders bevorzugte Lösungsmittel für die Verfahrensvariante b) sind Alkylnitrile mit 2 bis 5 Kohlenstoffatomen und 3-Alkoxypropionitrile mit 1 oder 2 C-Atomen im Alkoxyteil, besonders Acetonitril, Butyronitril, 3-Methoxypropionitril und Acrylnitril, während für die Verfahrensvariante a) auch aliphatische Alkohole mit bis zu 6 Kohlenstoffatomen, besonders Methanol und Aethanol, sowie überschüssiges Acrylnitril bevorzugt werden.

Die Reaktionstemperaturen für die Anlagerungsreaktionen liegen im allgemeinen zwischen etwa 70 und 220° C, bevorzugt zwischen etwa 90 und 130° C. Dabei kann in offenem oder geschlossenem Gefäss gearbeitet werden. Besonders bevorzugt wird die Anlagerung in geschlossenem System bei einem der jeweiligen Reaktionstemperatur entsprechenden Druck, der z.B. im Bereich von 0 bis 50 bar liegen kann, durchgeführt.

Die Ueberführung der 2,2,4-Trichlor-4-cyanoessigsäurealkylester der Formel II in Gegenwart von Ammoniak in das 2,2,4-Trichlor-4-cyano-butancarbonsäureamid wird auf an sich bekannte Weise, vorzugsweise in einem inerten organischen Lösungsmittel, besonders aliphatischen Alkoholen mit bis zu 6 Kohlenstoffatomen, vor allem Methanol oder Aethanol, vorgenommen. Die Umwandlung in das Amid erfolgt im allgemeinen bei einer Temperatur zwischen etwa 0 und 100° C, bevorzugt 0 bis 50° C.

Die Cyclisierung des 2,2,4-Trichlor-4-cyanobutancarbonsäureamids bzw. des 2,2,4-Trichlor-4-cyanobutyronitrils in wässrigem saurem Medium zum 3,3,5-Trichlorglutarsäureimid sowie dessen Ueberführung in das 2,3,5,6-Tetrachlorpyridin (Aromatisierung unter Dehydratisierung der Dicarbonsäureimidfunktionen und gleichzeitiger HCl-Abspaltung) können ebenfalls auf an sich bekannte Weise vorgenommen werden [vgl. z.B. Helv.Chim. Acta, 59, 179 (1976) und dort zitierte Literatur].Die Cyclisierung wird zweckmässig bei Temperaturen zwischen etwa -10 und +120° C, bevorzugt zwischen etwa 60 und 110° C und unter Zusatz von Essigsäure durchgeführt. Als Säuren können z.B. konzentrierte Salzsäure oder, bevorzugt, ca. 50-80%ige Schwefelsäure eingesetzt werden. Geeignete dehydratisierende Chlorierungsmittel für die Ueberführung des 3,3,5-Trichlorglutarsäureimids in das 2,3,5,6-Tetrachlorpyridin sind z.B. $PCl_3$, $PCl_5$, $POCl_3$, Phenylphosphorsäurechlorid und Phosgen. Bevorzugtes Chlorierungsmittel ist $POCl_3$. Unter Umständen empfiehlt sich der Zusatz katalytischer Mengen N,N-Dimethylformamid.

Die Reaktionstemperaturen liegen zweckmässig zwischen etwa 50 und 200° C und insbesondere zwischen etwa 100 und 180° C.

Die vorerwähnten neuen Zwischenprodukte sowie das 3,3,5-Trichlorglutarsäureimid und das 2,3,5,6-Tetrachlorpyridin können auf übliche Weise isoliert und gereinigt werden, beispielsweise durch Filtrieren, Destillieren, Umkristallisieren, etc.

Wie eingangs erwähnt, eignet sich das 2,3,5,6-Tetrachlorpyridin (und das daraus herstellbare 3,5,6-Trichlorpyridin-2-ol) zur Herstellung verschiedenartiger Wirkstoffe.

Beispiel 1 : Herstellung des 3,3,5-Trichlorglutarsäureimids der Formel

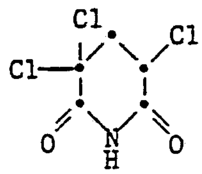

a) Zu 26,0 g 2,2,4-Trichlor-4-cyano-butancarbonsäureamid und 105 ml Eisessig werden 13 ml 50%ige Schwefelsäure gegeben und bei 65° C Badtemperatur so lange gerührt, bis Lösung eintritt (ca. 5 Minuten). Diese Reaktionslösung wird sofort auf Eis gegossen. Nach 1 Stunde Rühren wird filtriert. Man erhält 21,6 g 3,3,5-Trichlorglutarsäureimid; Smp. 158 bis 159° C. IR-Spektrum ($CHCl_3$): 1740 $cm^{-1}$ (CO); $^1H$-NMR-Spektrum ($CDCl_3$/DMSO-$d_6$) in ppm: 11,8 (breites s, mit $D_2O$ austauschbar, NH); 4,87 (X-Teil, J = 6 und 12 Hz, CH); 3,26 (AB-Teil, J = 6 und 12 und 15 Hz, $CH_2$).

Elementaranalyse für $C_5H_6Cl_3NO_2$ (Molgewicht 216,46):

| | | | | |
|---|---|---|---|---|
| berechnet | C 27,75 % | H 1,86 % | N 6,46 % | Cl 49,15 % |
| gefunden | C 27,78 % | H 1,84 % | N 6,52 % | Cl 49,21 %. |

Das im obigen Beispiel verwendete 2,2,4-Trichlor-4-cyanobutancarbonsäureamid wird wie folgt hergestellt: 300 ml Trichloressigsäureäthylester, 176 ml Acrylnitril, 7 g CuO und 500 ml Aethanol werden 20 Stunden auf 120° C gehalten. Das Reaktionsgemisch wird eingedampft, in Benzol aufgenommen, klarfiltriert und erneut eingedampft. Der erhaltene Rückstand wird destilliert. Man erhält 234,5 g 2,2,4-Trichlor-4-cyanoessigsäureäthylester; Sdp. 85° C/5 Pa. $^1H$-NMR-Spektrum ($CDCl_3$) in ppm: 4,93 (X-Teil, J = 6 und 7 Hz, CH); 4,4 (q, J = 7 Hz, $CH_2$); 3,35 (AB-Teil, J = 6 und 7 und 15 Hz. $CH_2$); 1,48 (t, J = 7 Hz, $CH_3$).

70,2 g des obigen Esters werden mit 84 ml Ammoniumhydroxidlösung (25%ig) und 150 ml Methanol bei 0° C zusammengegeben und 5 Stunden bei Raumtemperatur gerührt. Das Reaktionsgemisch wird auf 0° C abgekühlt und filtriert. Der Niederschlag wird mit Wasser gewaschen

und getrocknet. Man erhält 2,2,4-Trichlor-4-cyano-butancarbonsäure-amid; Smp. 178° C.

Elementaranalyse für $C_5H_5Cl_3N_2O$ (Molekulargewicht 201,47):

| | | | | |
|---|---|---|---|---|
| berechnet | C 28,0 % | H 2,35 % | Cl 49,2 % | N 13,05 % |
| gefunden | C 27,81% | H 2,37 % | Cl 49,29% | N 12,99 %. |

b) 20,0 g 2,2,4-Trichlor-4-cyanobutyronitril werden mit 70 ml Eisessig und 16 ml 78%iger Schwefelsäure 2 Stunden am Rückfluss gehalten. Die Reaktionslösung wird auf Eis gegossen und ausgerührt. Dann wird mit Methylenchlorid extrahiert. Nach dem Trocknen mit Magnesiumsulfat und dem Eindampfen des Extrakts wird der feste Rückstand mit Diäthyläther digeriert. Man erhält 3,3,5-Trichlorglutarsäureimid; Smp. 158-159° C, dessen spektroskopische Daten mit denjenigen des unter a) beschriebenen Imids identisch sind.

Das im obigen Beispiel verwendete 2,2,4-Trichlor-4-cyanobutyronitril wird wie folgt hergestellt: 53,1 g Acrylnitril, 433,2 g Trichloracetonitril, 7 g Kupfer(I)chlorid und 300 ml Acetonitril werden 24 Stunden in einem Email-Autoklaven auf 115° C gehalten. Das Reaktionsgemisch wird klärfiltriert und eingedampft. Der Rückstand wird destilliert. Man erhält 145 g 2,2,4-Trichlor-4-cyanobutyronitril; Sdp. 78°/65 Pa.

$^1$H-NMR-Spektrum ($CDCl_3$) in ppm: 4,87 (X-Teil, J = 6 und 6,5 Hz, CH); 3,30 (AB-Teil, J = 6 und 6,5 und 16 Hz, $CH_2$).

Elementaranalyse für $C_5H_3Cl_3N_2$ (Molgewicht 197,5):

| | | | | |
|---|---|---|---|---|
| berechnet | C 30,41 % | H 1,54 % | N 14,18 % | Cl 53,85 % |
| gefunden | C 29,9 % | H 1,6 % | N 13,8 % | Cl 54,1 %. |

Beispiel 2: 4,3 g 3,3,5-Trichlorglutarsäureimid und 80 ml Phosphoroxichlorid werden 12 Stunden bei 170° C gehalten . Das Reaktionsgemisch wird auf Eis gegossen und ausgerührt. Nach der Extraktion mit Benzol, dem Trocknen über Magnesiumsulfat und Behandlung mit Aktivkohle erhält man 3,65 g 2,3,5,6-Tetrachlorpyridin; Smp. 86-88° C.

Beispiel 3: 19,2 g 3,3,5-Trichlorglutarsäureimid und 100 ml Phosphoroxichlorid werden 3 Stunden auf 180° C gehalten. Das überschüssige Phosphoroxichlorid wird abdestilliert. Der Rückstand wird mit Wasserdampf destilliert. Nach dem Trocknen über Phosphoroxid erhält man 2,3,5,6-Tetrachlorpyridin; Smp. 89-90° C.

Beispiel 4: 12,4 g 3,3,5-Trichlorglutarsäureimid werden portionenweise in eine Lösung von 50 ml N,N-Dimethylformamid und 25 g $PCl_5$ eingetragen. Das Gemisch wird auf 100° C erwärmt, worauf man die Reaktionstemperatur bis auf 160° C steigert. Das erkaltete Reaktionsgemisch wird mit Eis zersetzt und 3 Stunden gerührt. Der Niederschlag wird abfiltriert, mit Wasser gewaschen und aus Methanol umkristallisiert. Man erhält 2,3,5,6-Tetrachlorpyridin; Smp. 86-88° C.

Patentansprüche

1. 3,3,5-Trichlorglutarsäureimid.

2. Ein Verfahren zur Herstellung von 3,3,5-Trichlorglutarsäureimid nach Anspruch 1, dadurch gekennzeichnet, dass man entweder

a) einen Trichloressigsäurealkylester der Formel I

$$Cl_3\,C\text{-}COOR \qquad (I),$$

worin R Alkyl mit 1 bis 6 Kohlenstoffatomen bedeutet, in Gegenwart eines Katalysators mit Acrylnitril zu einem 2,2,4-Trichlor-4-cyano-buttersäurealkylester der Formel II

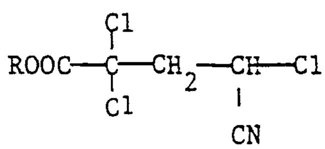

(II) ,

worin R die unter Formel I angegebene Bedeutung hat, umsetzt, die Verbindung der Formel II in Gegenwart von Ammoniak in das 2,2,4-Trichlor-4-cyano-butancarbonsäureamid überführt und das 2,2,4-Trichlor-4-cyano-butancarbonsäureamid in wässrigem saurem Medium zum 3,3,5-Trichlorglutarsäureimid cyclisiert oder

b) Trichloracetonitril in Gegenwart eines Katalysators mit Acrylnitril zum 2,2,4-Trichlor-4-cyanobutyronitril umsetzt und das 2,2,4-Trichlor-4-cyanobutyronitril in wässrigem saurem Medium zum 3,3,5-Trichlorglutarsäureimid cyclisiert.

3. Ein Verfahren nach Anspruch 2, dadurch gekennzeichnet, dass man die Umsetzung des Trichloressigsäurealkylesters der Formel I bzw. des Trichloracetonitrils mit dem Acrylnitril in Gegenwart eines organischen Lösungsmittels vornimmt.

4. Ein Verfahren nach Anspruch 2, dadurch gekennzeichnet, dass man für die Anlagerungsreaktion Kupfer(II)oxid, Kupferpulver, Kupferbronze, Kupfer(I)chlorid, Kupfer(II)chlorid, Kupfer(I)bromid,

Kupfer(II)bromid, Kupfer(I)jodid oder Gemische davon verwendet.

5. Ein Verfahren nach Anspruch 2, dadurch gekennzeichnet, dass man die Cyclisierung des 2,2,4-Trichlor-4-cyanobutancarbonsäureamids bzw. des 2,2,4-Trichlor-4-cyanobutyronitrils zum 3,3,5-Trichlorglutarsäureimid unter Zusatz von Essigsäure und etwa 50 bis 80%iger Schwefelsäure vornimmt.

6. Verwendung von 3,3,5-Trichlorglutarsäureimid nach Anspruch 1 zur Herstellung von 2,3,5,6-Tetrachlorpyridin, dadurch gekennzeichnet, dass man das 3,3,5-Trichlorglutarsäureimid mit einem dehydratisierenden Chlorierungsmittel behandelt.